(19) Europäisches Patentamt
European Patent Office
Office européen des brevets

(11) Veröffentlichungsnummer: **0 233 568**
**B1**

(12) # EUROPÄISCHE PATENTSCHRIFT

(45) Veröffentlichungstag der Patentschrift: **09.08.89**

(51) Int. Cl.⁴: **C07C 49/753**, C07C 49/757, C07C 45/54, C07C 45/71, C07C 45/51, A01N 35/06

(21) Anmeldenummer: **87101738.0**

(22) Anmeldetag: **09.02.87**

(54) **Cyclohexenonderivate, Verfahren zu ihrer Herstellung und ihre Verwendung als Mittel zur Regulierung des Pflanzenwachstums.**

(30) Priorität: **15.02.86 DE 3604871**

(43) Veröffentlichungstag der Anmeldung: **26.08.87 Patentblatt 87/35**

(45) Bekanntmachung des Hinweises auf die Patenterteilung: **09.08.89 Patentblatt 89/32**

(84) Benannte Vertragsstaaten: **BE CH DE FR GB IT LI NL**

(56) Entgegenhaltungen:
**EP-A- 0 123 001**

**SYNTHESIS, December 1978, Seiten 925-927, Georg Thieme Publishers; A.A. AKHREM et al.: "A new simple synthesis of 2-acylcyclohexane-1,3-diones"**

(73) Patentinhaber: **BASF Aktiengesellschaft, Carl-Bosch-Strasse 38, D-6700 Ludwigshafen(DE)**

(72) Erfinder: **Keil, Michael, Dr., Fontanestrasse 4, D-6713 Freinsheim(DE)**
Erfinder: **Schirmer, Ulrich, Dr., Berghalde 79, D-6900 Heidelberg(DE)**
Erfinder: **Kolassa, Dieter, Dr., Moltkestrasse 8, D-6700 Ludwigshafen(DE)**
Erfinder: **Rademacher, Wilhelm, Dr., Austrasse 1, D-6703 Limburgerhof(DE)**
Erfinder: **Jung, Johann, Prof. Dr., Hardenburgstrasse 19, D-6703 Limburgerhof(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

EP 0 233 568 B1

## Beschreibung

Die Erfindung betrifft neue Cyclohexenonderivate, Verfahren zu ihrer Herstellung und ihre Verwendung als Mittel, die das Wachstum von Pflanzen regulieren sowie ein Verfahren zur Regulierung des Pflanzenwachstums.

Es ist bekannt, daß bestimmte 2-Acyl-3-hydroxycyclohex-2-en-1-one das Pflanzenwachstum beeinflussen (EP-A-123 OO1, EP-A-126 713).

Es wurde nun gefunden, daß Cyclohexenonderivate der Formel I

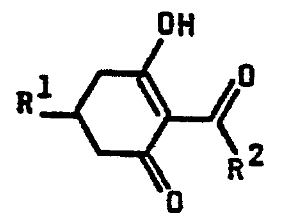

I,

in der

$R^1$ -CHO oder -CH(OR$^3$)$_2$

$R^2$ Alkyl, Alkoxyalkyl mit jeweils 1 bis 4 Kohlenstoffatomen oder Cyclopropyl, und

$R^3$ Alkyl mit 1 bis 8 Kohlenstoffatomen bedeuten,

oder Salze dieser Verbindungen vorteilhafte Eigenschaften als Wachstumsregulatoren und auch hinsichtlich der Verträglichkeit für Pflanzen aufweisen.

$R^1$ bedeutet beispielsweise Formyl, Dimethoxymethyl, Diethoxymethyl, Dibutoxymethyl,

$R^2$ steht für Methyl, Ethyl, n-Propyl, i-Propyl, n-Butyl, sec-Butyl, i-Butyl, tert-Butyl oder die entsprechenden Alkoxysubstituenten oder für Cyclopropyl.

Als Salze der Verbindungen der Formel I kommen landwirtschaftlich brauchbare Salze, beispielsweise Alkalimetallsalze, insbesondere Kalium- oder Natriumsalze, Erdalkalimetallsalze, insbesondere Calcium-Magnesium- oder Bariumsalze, Mangan-, Kupfer-, Zink- oder Eisensalze sowie Ammonium-, Phosphonium-, Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoxoniumsalze in Betracht.

Die Cyclohexenonderivate der Formel I können durch Umsetzung einer entsprechenden Verbindung der Formel II

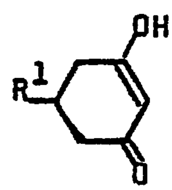

II,

in üblicher Weise mit einem Säurechlorid $R^2$-COCl, z.B. mittels Triethylamin, Pyridin, Natriumhydroxid, Kaliumcarbonat zum Enolester und anschließende Umlagerung mit einer Imidazol- oder Pyridinbase, beispielsweise 4-N,N-Dimethylaminopyridin erhalten werden.

Hat man auf diese Weise zunächst ein Acetal, d.h. eine Verbindung mit der Bedeutung CH(OR$^3$)$_2$ für $R^1$ hergestellt, so kann man natürlich mittels einer Säure wie Salzsäure, Schwefelsäure, Phosphorsäure, Ameisensäure, Essigsäure, Oxalsäure in einem Lösungsmittel wie z.B. Tetrahydrofuran, 1,4-Dioxan, Dichlormethan, Methanol, Ethanol, Toluol den freien Aldehyd ($R^1$ = -CHO) gewinnen.

Ebenso kann man zunächst ein entsprechendes Thioacetal herstellen und dieses verseifen nach

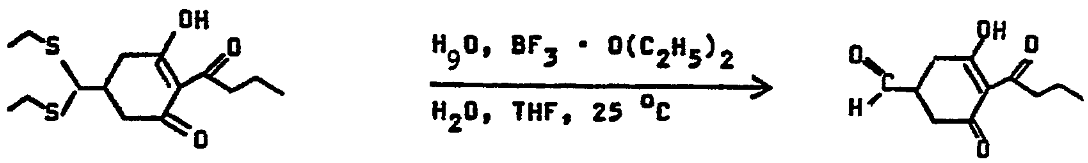


Diese Umsetzung gelingt z.B. in Gegenwart eines Quecksilbersalzes und Borfluorid-Etherat, wie vorstehend angegeben..

Die für die Umsetzung benötigten Vorprodukte lassen sich nach dem nachstehenden Schema gewinnen:

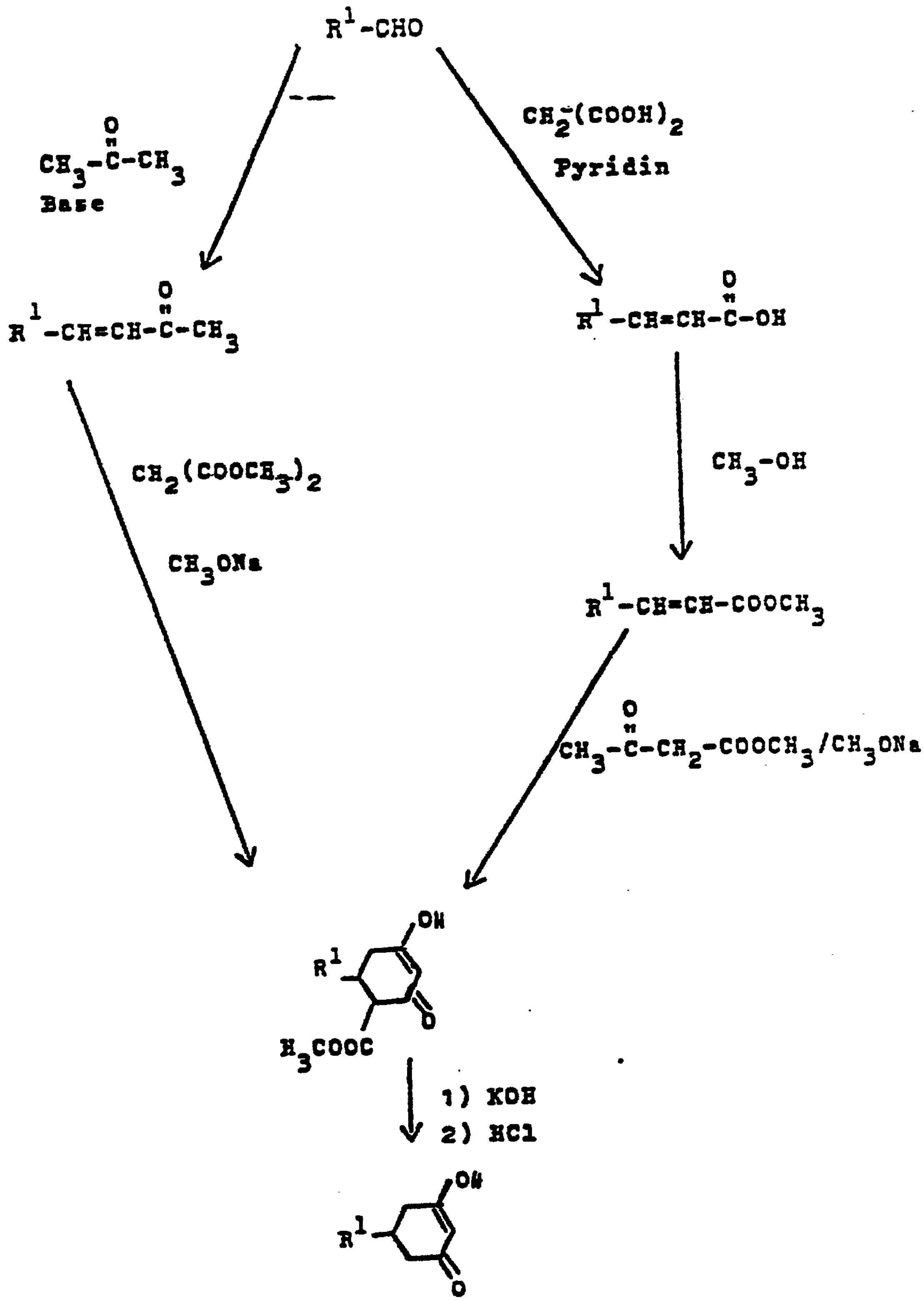


Die zur Charakterisierung geeigneten 1H-NMR-Spektren der nachstehenden Verbindungen wurden in Deuterochloroform als Lösungsmittel mit Tetramethylsilan als internem Standard aufgenommen. Die 1H-

chemischen Verschiebungen sind jeweils in (ppm) angegeben, wobei nur charakteristische Signale genannt sind. Für die Signalstruktur gelten folgende Abkürzungen:
s = Singulett, d = Dublett, t = Triplett, q = Quartett, m = Multiplett.

Beispiel 1

3,16 g 5- Bis(ethylthio)-methyl -2-butyryl-3-hydroxy-cyclohex-2-en-1-on in 1O ml Tetrahydrofuran wird zu einer Mischung von 4,33 g Quecksilber(II)-oxid, 2,84 g Bortrifluoridetherat, 5O ml Tetrahydrofuran und 33O ml Wasser getropft. Nach 3O Min. wird filtriert, 2 mal mit Diethylether extrahiert, eingeengt und über Kieselgel chromatographiert. Man erhält 3,O g (86 % d. Th.) 2-Butylryl-5-formyl-3-hydroxy-cyclohex-2-en-1-on.

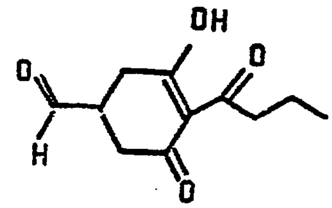

(Verbindung Nr. 1 der nachstehenden Tabelle)
¹H-NMR: O,98 (t); 1,65 (m); 2,55 - 2,95 (m); 3,O1 (t); 9,77 (s); 16,7 (s).

Beispiel 2

1,1 g 2-Butyrl-5-formyl-3-hydroxy-cyclohex-2-en-1-on, O,6 g Trimethylorthoformiat, 5O ml Methanol und O,O5 g saurer Ionenaustauscher (z.B. Amberlyst 15) werden 2 Stunden bei Rückfluß erhitzt, nach dem Abkühlen mit 1OO ml Diethylether verdünnt und über eine dünne Schicht Kieselgel filtriert. Das Filtrat wird eingeengt, es verbleibt O,9 g 5-Bis(methoxy)-methyl-2-butyryl-3-hydroxy-cyclohex-2-en-1-on als Öl.

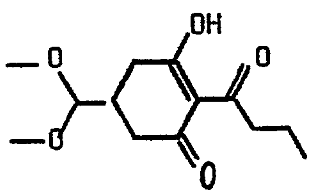

(Verbindung Nr. 8 der nachstehenden Tabelle)

Tabelle

| Nr. | $R^1$ | $R^2$ | Charakteristische $^1H$–NMR-Daten bzw. Schmelzpunkte |
|---|---|---|---|
| 1 | $-CHO$ | $n-C_3H_7$ | 0,98 (t); 1,65 (m); 3,01 (t); 9,77 (s); 16,7 (s) |
| 2 | $-CHO$ | $C_2H_5$ | 1,17 (t); 3,09 (q); 9,73 (s) |
| 3 | $-CHO$ | $CH_3$ | |
| 4 | $-CHO$ | $n-C_4H_9$ | |
| 5 | $-CHO$ | $i-C_3H_7$ | |
| 6 | $-CH(OCH_3)_2$ | $CH_3$ | |
| 7 | $-CH(OCH_3)_2$ | $C_2H_5$ | 1,25 (t); 3,12 (q); 3,40 (s) |
| 8 | $-CH(OCH_3)_2$ | $n-C_3H_7$ | 0,97 (t); 3,40 (s); 4,12 (d) |
| 9 | $-CH(OCH_3)_2$ | $i-C_3H_7$ | |
| 10 | $-CH(OCH_3)_2$ | $n-C_4H_9$ | |
| 11 | $-CH(OC_2H_5)_2$ | $C_2H_5$ | |
| 12 | $-CH(OC_2H_5)_2$ | $n-C_3H_7$ | 0,98 (t); 3,71 (q); 4,30 (d) |
| 13 | $-CH(OC_4H_9)_2$ | $C_2H_5$ | |
| 14 | $-CH(OC_4H_9)_2$ | $-n-C_3H_7$ | 2,99 (t); 3,62 (t); 4,30 (d) |
| 15 | $-CHO$ Natriumsalz | $n-C_3H_7$ | 92–95°C |
| 16 | $-CHO$ Kaliumsalz | $n-C_3H_7$ | 140°C (Zers.) |
| 17 | $-CHO$ Tetrabutylammoniumsalz | $n-C_3H_7$ | |
| 18 | $-CH(OCH_3)_2$ Natriumsalz | $n-C_3H_7$ | 160°C (Zers.) |
| 19 | $-CH(OCH_3)_2$ Kaliumsalz | $n-C_3H_7$ | 80°C (Zers.) |
| 20 | $-CH(OCH_3)_2$ Tetrabutylammoniumsalz | $n-C_3H_7$ | 0,93 (t); 3,23 (s); 4,08 (d) |
| 21 | $-CHO$ | Cyclopropyl | |
| 22 | $-CHO$ | Methoxyethyl | |
| 23 | $-CHO$ | $C_2H_5$ | 110–112 °C |
| 24 | $-CHO$ Kaliumsalz | $C_2H_5$ | 127–129 °C |
| 25 | $-CH(OCH_3)_2$ Natriumsalz | $C_2H_5$ | 103–114 °C |

Die Cyclohexenonderivate der Formel I können praktisch alle Entwicklungsstadien einer Pflanze verschiedenartig beeinflussen und werden deshalb als Wachstumsregulatoren eingesetzt.

Zur Bestimmung der wachstumsregulierenden Eigenschaft der Prüfsubstanzen wurden Testpflanzen auf ausreichend mit Nährstoffen versorgtem Kultursubstrat in Kunststoffgefäßen (ca. 12,5 cm Durchmesser; Volumen ca. 500 ml) angezogen.

Bei Bodenbehandlungen wurden die Testsubstanzen in wäßriger Aufbereitung entweder am Tage der Einsaat (Vorauflaufverfahren) oder nach erfolgter Keimung (Nachauflaufverfahren) auf das Substrat gegossen.

Bei Blattapplikationen wurden die zu prüfenden Substanzen in wäßriger Aufbereitung auf die Pflanzen gesprüht (Nachauf-Blattbehandlung).

Als Vergleichssubstanz diente der bekannte, handelsübliche Wachstumsregulator 2-Chlorethylmethylammoniumchlorid (Chlormequatchlorid, CCC).

Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Die Einzeldaten sind den folgenden Tabellen zu entnehmen:

Tabelle 1

Sommerweizen, Sorte «Kolibri», Nachauflauf-Blattbehandlung

| Nr. d. chem. Beispiele | Konz. mg WS/Gef. | Wuchshöhen rel. |
|---|---|---|
| unbehandelt | – | 100 |
| CCC | 6 | 69,0 |
| 1 | 6 | 64,4 |
| 2 | 6 | 61,8 |
| 7 | 6 | 75,5 |
| 8 | 6 | 67,6 |
| 15 | 6 | 86,4 |
| 16 | 6 | 69,2 |
| 18 | 6 | 92,7 |
| 19 | 6 | 75,4 |
| 20 | 6 | 81,7 |

Tabelle 2

Sommergerste, Sorte «Aramir», Nachauflauf-Blattbehandlung

| Nr. d. chem. Beispiele | Konz. mg WS/Gef. | Wuchshöhen rel. |
|---|---|---|
| unbehandelt | – | 100 |
| CCC | 6 | 79,6 |
| 1 | 6 | 58,9 |
| 2 | 6 | 73,6 |
| 7 | 6 | 78,4 |
| 8 | 6 | 68,7 |
| 15 | 6 | 92,5 |
| 16 | 6 | 71,6 |
| 18 | 6 | 86,6 |
| 19 | 6 | 73,1 |
| 20 | 6 | 88,1 |

Tabelle 3

Sommergerste, Sorte «Aramir», Vorauflauf-Bodenbehandlung

| Nr. d. chem. Beispiele | Konz. mg WS/Gef. | Wuchshöhen rel. |
|---|---|---|
| unbehandelt | – | 100 |
| CCC | 6 | 80,8 |
| 1 | 6 | 60,1 |
| 2 | 6 | 53,5 |
| 23 | 6 | 76,6 |

Tabelle 4

| Reis «Nihonbare», Nachauflauf-Blattbehandlung | | |
|---|---|---|
| Nr. d. chem. Beispiele | Konz. mg WS/Gef. | Wuchshöhen rel. |
| unbehandelt | - | 100 |
| CCC | 1,5 | 99,8 |
| | 6 | 99,8 |
| 1 | 1,5 | 72,3 |
| | 6 | 37,9 |

## Patentansprüche

1. Cyclohexenonderivate der Formel

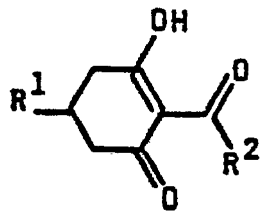

I,

in der
$R^1$ -CHO oder $-CH(OR^3)_2$

$R^2$ Alkyl, Alkoxyalkyl mit jeweils 1 bis 4 Kohlenstoffatomen oder Cyclopropyl und

$R^3$ Alkyl mit jeweils 1 bis 8 Kohlenstoffatomen bedeuten,

oder Salze dieser Verbindungen.

2. Verfahren zur Herstellung eines Cyclohexenonderivates der Formel I nach Anspruch 1, dadurch gekennzeichnet, daß man eine entsprechende Verbindung der Formel II

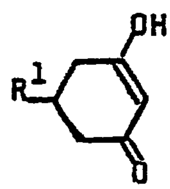

II,

mit einem Säurechlorid $R^2COCl$ und einer Base in den Enolester überführt und diesen in Gegenwart von Imidazol- oder Pyridinderivaten umlagert.

3. Verfahren zur Herstellung eines Cyclohexenonderivates der Formel I nach Anspruch 1 mit der Bedeutung $R^1$ = -CHO, dadurch gekennzeichnet, daß man das entsprechende Acetal mit einer Säure oder das Thioacetal mit einem Quecksilbersalz spaltet.

4. Verfahren zur Herstellung eines Cyclohexenonderivates der Formel I nach Anspruch 1 mit der Bedeutung $R^1$ = $-CH(OR^3)_2$ dadurch gekennzeichnet, daß man den entsprechenden Aldehyd in an sich bekannter Weise in das Acetal überführt.

5. Mittel zur Regulierung des Pflanzenwachstums, enthaltend mindestens ein Cyclohexenonderivat der Formel I nach Anspruch 1 oder ein Salz dieser Verbindung.

6. Mittel zur Regulierung des Pflanzenwachstums enthaltend mindestens ein Cyclohexenonderivat der Formel I nach Anspruch 1 und einen flüssigen oder festen Trägerstoff sowie gegebenenfalls ein oder mehrere oberflächenaktive Mittel.

7. Verfahren zur Regulierung des Pflanzenwachstums, dadurch gekennzeichnet, daß man mindestens ein Cyclohexenonderivat der Formel I nach Anspruch 1 auf Pflanzen oder deren Lebensraum einwirken läßt.

## Claims

1. A cyclohexenone derivative of the formula

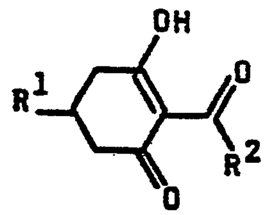

I,

where $R^1$ is $-CHO$ or $-CH(OR^3)_2$, $R^2$ is alkyl or alkoxyalkyl, each of 1 to 4 carbon atoms, or cyclopropyl and $R^3$ is alkyl of 1 to 8 carbon atoms, or a salt of this compound.

2. A process for the preparation of a cyclohexenone derivative of the formula I as claimed in claim 1, wherein a corresponding compound of the formula II

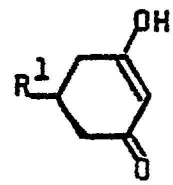

II,

is converted with an acyl chloride $R^2COCl$ and a base into the enolester and the latter is subjected to a rearrangement reaction in the presence of an imidazole or pyridine derivative.

3. A process for the preparation of a cyclohexenone derivative of the formula I as claimed in claim 1 where $R^1$ is $-CHO$, wherein the corresponding acetal is cleaved with an acid or the thioacetal is cleaved with a mercury salt.

4. A process for the preparation of a cyclohexenone derivative of the formula I as claimed in claim 1 where $R^1$ is $-CH(OR^3)_2$, wherein the corresponding aldehyde is converted into the acetal in a conventional manner.

5. A plant growth regulator containing one or more cyclohexenone derivatives of the formula I as claimed in claim 1 or a salt of this compound.

6. A plant growth regulator containing one or more cyclohexenone derivatives of the formula I as claimed in claim 1 and a liquid or solid carrier and, if required, one or more surfactants.

7. A method for regulating plant growth, wherein one or more cyclohexenone derivatives of the formula I as claimed in claim 1 are allowed to act on plants or their habitat.

## Revendications

1. Dérivés de cyclohexenone de formule

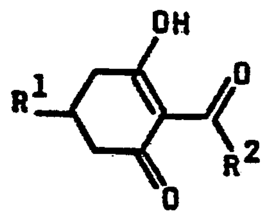

I,

dans laquelle $R^1$ représente $-CHO$ ou $-CH(OR^3)_2$, $R^2$, alkyle, alcoxyalkyle ayant chacun 1 à 4 atomes de carbone ou cyclopropyle et $R^3$ représente alkyle ayant 1 à 8 atomes de carbone ou des sels de ces composés.

2. Procédé de préparation de dérivés de cyclohexenone de formule I selon la revendication 1, caractérisé par le fait que l'on transforme un composé correspondant de formule II

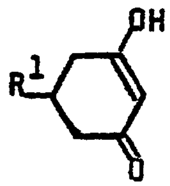

II,

avec un chlorure d'acide $R^2COCl$ et une base, en l'ester énolique et on transpose celui-ci en présence de dérivés d'imidazole ou de pyridine.

3. Procédé de préparation d'un dérivé de cyclohexenone de formule I selon la revendication 1, avec la signification $R^1$ = –CHO, caractérisé par le fait que l'on scinde l'acétal correspondant avec un acide ou le thiacétal avec un sel de mercure.

4. Procédé de préparation d'un dérivé de cyclohexenone avec la signification $R^1 = -CH(OR^3)_2$, caractérisé par le fait que l'on transforme de manière connue en soi, l'aldéhyde correspondant en l'acétal.

5. Moyen de régulation de la croissance des plantes, contenant au moins un dérivé de cyclohexenone de formule I selon la revendication 1 ou un sel ce composé.

6. Moyen de régulation de la croissance des plantes, contenant au moins un dérivé de cyclohexenone de formule I selon la revendication 1 et un support solide ou liquide ainsi qu'éventuellement un ou plusieurs agents tensio-actifs.

7. Procédé de régulation de la croissance des plantes, caractérisé par le fait que l'on fait agir sur les plantes ou leur biotope un dérivé de cyclohexenone de formule I selon la revendication 1.